# EUROPÄISCHE PATENTSCHRIFT

(11) **EP 3 224 682 B1**
(45) Veröffentlichungstag und Bekanntmachung des Hinweises auf die Patenterteilung: **18.09.2024**
(21) Anmeldenummer: 15808359.2
(22) Anmeldetag: 20.11.2015
(51) Int. Cl.: G16C 20/10, G16C 20/40, G05B 19/418, G05B 23/02

(54) **VERFAHREN ZUR KONFIGURATION EINER ZUR DURCHFÜHRUNG VON WENIGSTENS EINER CHEMISCHEN REAKTION EINGERICHTETEN PRODUKTIONSANLAGE**
METHOD FOR CONFIGURING A PRODUCTION PLANT DESIGNED FOR PERFORMING AT LEAST ONE CHEMICAL REACTION
PROCÉDÉ POUR LA CONFIGURATION D'UNE INSTALLATION DE PRODUCTION CONÇUE POUR LA RÉALISATION D'AU MOINS UNE RÉACTION CHIMIQUE

(30) Priorität: 24.11.2014 DE 102014117122
(43) Veröffentlichungstag der Anmeldung: 04.10.2017
(73) Patentinhaber: Suzhou Skywell Healthcare Information Co., Ltd., Jiangsu 215021 (CN)
(72) Erfinder: FLEISCHER-TREBES, Christoph, 45131 Essen (DE); BRÖTZ, Bojan Niko, 51381 Leverkusen (DE)
(74) Vertreter: Simmons & Simmons LLP (Munich)
(86) Internationale Anmeldenummer: PCT/EP2015/077243
(87) Internationale Veröffentlichungsnummer: WO 2016/083262

(56) Entgegenhaltungen:
- US-A1- 2007 078 540
- ABB AUTOMATION: "System 800xA - The Power of Integration: 800xA Device Management and Fieldbus Overview", 31 December 2010 (2010-12-31), pages 1 - 16, XP055082479, Retrieved from the Internet <URL:http://www05.abb.com/global/scot/scot349.nsf/veritydisplay/4b7d270eb6928987c1257837004a37a3/$file/3bdd013081_l_d_en_system_800xa_5.1_device_management_overview.pdf> [retrieved on 20131003]

## Beschreibung

Die vorliegende Erfindung betrifft ein Verfahren zur Konfiguration einer zur Durchführung von wenigstens einer chemischen Reaktion eingerichteten Produktionsanlage nach Anspruch 1. Bevorzugte Ausgestaltungen der Erfindung sind in den abhängigen Ansprüchen definiert.

Des Weiteren betrifft die Erfindung ein Computerprogramm mit auf einem computerlesbaren Datenträger gespeicherten Programmcodemitteln nach Anspruch 8 und einen Datenträger nach Anspruch Für die Durchführung einer chemischen Reaktion zur Herstellung eines bestimmten chemischen Produkts ist es erforderlich, eine Produktionsanlage mit individuell gestalteter Anlagenstruktur bereitzustellen. Um die jeweilig verfahrenstechnisch erforderlichen Prozessschritte in einzelnen Anlagenmodulen durchführen zu können, sind hierzu technisch geeignete, eine Mehrzahl von Bauteilkomponenten aufweisende, Anlagenmodule miteinander zu einer Produktionsanlage zu kombinieren. Die Veröffentlichung US 2007 007 8540 A1 offenbart vernetzte computergestützte industrielle Prozesssteuerungssysteme und Dienstprogramme für die Unterstützung von Feldgeräten.

Üblicherweise ist in einem Laborlager eine Vielzahl von Anlagenmodulen verfügbar, wobei in der Regel davon ausgegangen werden kann, dass keines dieser Anlagenmodule aus dem Bestand die prozessspezifischen und zum Teil sehr komplexen technischen Anforderungen zur Herstellung eines bestimmten chemischen Produkts direkt erfüllt. Es ist daher sehr schwierig ein im Bestand vorhandenes Anlagenmodul erneut zu verwenden, so dass in der Regel ein neues, die den bestimmten komplexen technischen Anforderungen erfüllendes, Anlagenmodul aus einer Vielzahl von Bauteilkomponenten zusammengestellt werden muss. Da jede Neuentwicklung von Anlagenmodulen mit Mehraufwand im Vergleich zur 1:1 Wiederverwendung von Anlagenmodulen verbunden ist und dadurch der zeitreduzierende Effekt bei der Planung modularer Produktionsanlagen ausbleibt, sollte die Auswahl sowie Konfiguration neuer Anlagenmodule im Hinblick auf den zu realisierenden Prozess beschleunigt werden.

Es ist daher die Aufgabe der Erfindung, eine zügige und preiswerte Konfiguration einer zur Durchführung von wenigstens einer chemischen Reaktion eingerichteten Produktionsanlage bereitzustellen.

Diese Aufgabe wird durch ein Verfahren mit den Merkmalen gemäß Patentanspruch 1, ein Computerprogramm mit den Merkmalen gemäß Patentanspruch 8 sowie einen Datenträger mit den Merkmalen gemäß Patentanspruch 9 gelöst. Bevorzugte Ausgestaltungen sind in den Unteransprüchen angegeben, die jeweils für sich genommen oder in beliebiger Kombination miteinander einen Aspekt der Erfindung darstellen können.

Gemäß Anspruch 1 wird ein Verfahren zur Konfiguration einer zur Durchführung von wenigstens einer chemischen Reaktion eingerichteten Produktionsanlage bereitgestellt, wobei
die Produktionsanlage wenigstens ein Anlagenmodul aufweist, und zur Konfiguration der Produktionsanlage ein Anlagenmodul aus einer auf einem ersten Serversystem laufenden Moduldatenbank ausgewählt und/oder aus einer auf einem zweiten Serversystem laufenden Komponentendatenbank zusammengestellt wird, und
das Auswählen und/oder Zusammenstellen eines Anlagenmoduls folgende Schritte umfasst:
   - Manuelle erste Eingabe prozessspezifischer technischer Anforderungen an ein Anlagenmodul in eine Abfragemaske der Moduldatenbank und Bestätigung der Eingabe;
   - Abgleich der ersten Eingabe mit wenigstens einem in der Moduldatenbank hinterlegten, eine prozessspezifische Eigenschaft eines jeweiligen Anlagenmoduls definierenden, technischen Parameter der Vielzahl von Anlagenmodulen auf dem ersten Serversystem, und bei einem negativem Ergebnis:
   - Ausweisen wenigstens eines Anlagenmoduls dessen prozessspezifische Eigenschaft die prozessspezifischen technischen Anforderungen nicht erfüllt in einer Ausgabemaske der Moduldatenbank;
   - Identifikation wenigstens einer die Anforderungen an das Anlagenmodul nicht erfüllenden Bauteilkomponente des ausgewiesenen Anlagenmoduls und/oder eines die Anforderungen an das Anlagenmodul nicht erfüllenden technischen Parameters einer Baureihenkomponente der Bauteilkomponente des ausgewiesenen Anlagenmoduls;
   - Manuelle und/oder automatische zweite Eingabe prozessspezifischer technischer Anforderungen für die identifizierte Bauteilkomponente und/oder des technischen Parameters der Baureihenkomponente in eine Abfragemaske der Komponentendatenbank;
   - Abgleich der zweiten Eingabe mit den in der Komponentendatenbank hinterlegten, die prozessspezifische Eigenschaft einer jeweiligen Baureihenkomponente definierenden, technischen Parameter der Vielzahl von Baureihenkomponenten, und bei positivem Ergebnis:
   - Ausweisen wenigstens einer die prozessspezifischen technischen Anforderungen erfüllenden Baureihenkomponente einer Bauteilkomponente in einer Ausgabemaske der Komponentendatenbank und Zusammenstellen des ausgegeben Anlagenmoduls mit der ausgegebenen Baureihenkomponente.

Die Moduldatenbank weist eine Vielzahl von Anlagenmodulen auf und jedes Anlagenmodul umfasst eine Vielzahl von Bauteilkomponenten, wobei jede Bauteilkomponente eine Baureihenkomponente mit wenigstens einem, vorzugsweise mehreren, die prozessspezifische Eigenschaft der Baureihenkomponente definierenden technischen Parameter aufweist. Die prozessspezifische Eigenschaft eines Anlagenmoduls umfasst wenigstens einen, vorzugsweise mehrere, die prozessspezifische Eigenschaft einer Baureihenkomponente definierenden technischen Parameter. Auf diese Weise kann die prozessspezifische Eigenschaft eines in der Moduldatenbank hinterlegten Anlagenmoduls eindeutig definiert werden.

Die Komponentendatenbank weist eine Vielzahl von Bauteilkomponenten mit jeweils wenigstens einer Baureihenkomponente auf, wobei jeder Baureihenkomponente wenigstens ein, vorzugsweise mehrere, eine prozessspezifische Eigenschaft einer jeweiligen Baureihenkomponente definierender technischer Parameter zugeordnet ist.

Unter prozessspezifischen technischen Anforderungen an ein Anlagenmodul wird ein technischer Parameter verstanden der von dem Anlagenmodul und/oder von einer Bauteilkomponente des Anlagenmoduls erfüllt sein sollte bzw. erfüllt sein muss, um vorzugsweise eine chemische Reaktion in dem Anlagenmodul sicher durchführen zu können. Besonders bevorzugt sind dies jedoch mehrere technische Parameter, wobei ein erster technischer Parameter vorzugsweise eine bauteilkomponentenspezifische Größe einer Bauteilkomponente ist und/oder ein zweiter technischer Parameter eine baureihenspezifische Größe einer Baureihenkomponente ist. Vorzugsweise sind der erste technische Parameter und/oder der zweite technische Parameter Angaben bzw. Werte zur Durchflussmenge und/oder zum Betriebsdruck und/oder zur Betriebstemperatur und/oder zur Verweilzeit eines Stoffes in einer Bauteilkomponente bzw. in der Mehrzahl der Bauteilkomponenten eines Anlagenmoduls. Die Einheit, in der die technischen Parameter in die Abfragemaske der Moduldatenbank und/oder der Abfragemaske der Komponentendatenbank eingegeben werden, ist von der Abfragemaske der Moduldatenbank bzw. der Abfragemaske der Komponentendatenbank vorgegeben.

Die erste Eingabe und/oder die zweite Eingabe erfolgt vorzugsweise über ein Eingabemodul, wobei das Eingabemodul mit dem ersten Serversystem und/oder dem zweiten Serversystem kommunikationstechnisch verbunden ist. Vorzugsweise ist das Eingabemodul ein Computer und/oder ein Laptop und/oder ein Tablet.

Die Abfragemaske der Moduldatenbank und/oder die Ausgabemaske der Moduldatenbank und/oder die Abfragemaske der Komponentendatenbank und/oder die Ausgabemaske der Komponentendatenbank sind auf dem Eingabemodul darstellbar. Vorzugsweise erfolgt die Darstellung in Tabellenform auf einem Bildschirm und/oder einem Monitor des Eingabemoduls.

Ein Aspekt der Erfindung ist somit, dass in einem ersten Schritt nach einem die prozessspezifischen technischen Anforderungen erfüllendem Anlagenmodul in der Moduldatenbank gesucht wird. Sofern kein die prozessspezifischen technischen Anforderungen erfüllendes Anlagenmodul verfügbar ist, wird wenigstens ein Anlagenmodul ausgewiesen, dass die prozessspezifischen technischen Anforderungen nicht erfüllt, wobei die die Anforderungen nicht erfüllende Bauteilkomponente und/oder der die Anforderungen nicht erfüllende technische Parameter einer Baureihenkomponente der Bauteilkomponente des ausgewiesenen Anlagenmoduls identifiziert und ausgewiesen wird. Auf diese Weise kann für die ausgewiesene und die Anforderungen nicht erfüllende Bauteilkomponente in der Komponentendatenbank nach einer die Anforderungen erfüllenden Baureihenkomponente der Bauteilkomponente gesucht werden. Sofern eine die Anforderungen erfüllende Baureihenkomponente in der Komponentendatenbank verfügbar ist, kann das ausgewiesene Anlagenmodul durch den Austausch der die Anforderungen nicht erfüllenden Bauteilkomponente mit der in der Komponentendatenbank ausgewiesenen, die Anforderungen erfüllende Baureihenkomponente der Bauteilkomponente, angepasst werden. Auf diese Weise wird ein Verfahren bereitgestellt, das eine zügige und preiswerte Konfiguration der zur Durchführung von wenigstens einer chemischen Reaktion eingerichteten Produktionsanlage ermöglicht.

Eine bevorzugte Weiterentwicklung der Erfindung sieht vor, dass nach einem positiven Abgleich der prozessspezifischen technischen Anforderungen an ein Anlagenmodul mit den in der Moduldatenbank hinterlegten technischen Parametern der Vielzahl von Anlagenmodulen der Schritt erfolgt:
- Ausweisen wenigstens eines Anlagenmoduls dessen prozessspezifische Eigenschaft die prozessspezifischen technischen Anforderungen erfüllt.

Auf diese Weise kann bereits nach dem ersten Prüfschritt ein die prozessspezifischen technischen Anforderungen erfüllendes Anlagenmodul ausgewiesen werden, wodurch die Konfiguration der zur Durchführung von wenigstens einer chemischen Reaktion eingerichteten Produktionsanlage beschleunigt werden kann. Sofern ein die prozessspezifischen technischen Anforderungen erfüllendes Anlagenmodul bereits nach dem ersten Prüfschritt ausgewiesen wird, sind die folgenden Prüfschritte optional. Es kann somit zudem ein die prozessspezifischen Anforderungen nicht erfüllendes Anlagenmodul ausgewiesen werden.

Gemäß einer weiteren bevorzugten Weiterentwicklung der Erfindung ist vorgesehen, dass nach einem negativen Abgleich der manuellen und/oder automatischen zweiten Eingabe der prozessspezifischen technischen Anforderungen für die identifizierte Bauteilkomponente und/oder des technischen Parameters der Baureihenkomponente mit den in der Komponentendatenbank hinterlegten technischen Parameter der Vielzahl von Baureihenkomponenten der Schritt erfolgt:
- Manuelle und/oder automatische dritte Eingabe prozessspezifischer technischer Anforderungen an ein Anlagenmodul in die Abfragemaske der Komponentendatenbank;
- Abgleich prozessspezifischer technischer Anforderungen an das Anlagenmodul mit den in der Komponentendatenbank hinterlegten, die prozessspezifische Eigenschaft einer jeweiligen Baureihenkomponente definierenden, technischen Parameter der Vielzahl von Baureihenkomponenten der Bauteilkomponenten;
- Ausgabe einer Vielzahl von Bauteilkomponenten, dessen prozessspezifische Eigenschaften der Baureihenkomponenten die prozessspezifischen technischen Anforderungen an das Anlagenmodul erfüllen;
- Bereitstellen eines die prozessspezifischen technischen Anforderungen erfüllendes Anlagenmodul aus der Vielzahl der ausgegeben Bauteilkomponenten und/oder Baureihenkomponenten.

Auf diese Weise wird aus einer Vielzahl von Baureihenkomponenten unterschiedlicher Bauteilkomponenten ein die prozessspezifischen technischen Anforderungen erfüllendes Anlagenmodul neu konfiguriert. Die Konfiguration eines neuen Anlagenmoduls aus einzelnen Bauteilkomponenten erfolgt vorzugsweise erst nachdem geprüft wurde, ob ein die prozessspezifischen technischen Anforderungen nicht erfüllendes Anlagenmodul mit wenigstens einer aus der Komponentendatenbank ausgewiesenen Bauteilkomponente derart zusammengestellt werden kann, dass es die prozessspezifischen technischen Anforderungen an das Anlagenmodul erfüllt. Somit kann sichergestellt werden, dass zunächst derartige vorhandene Anlagenmodule ausgewiesen werden, die mit geringem Aufwand umgebaut werden könnten, wodurch ein die prozessspezifischen technischen Anforderungen erfüllendes Anlagenmodul zügig und mit geringen Kosten bereitgestellt werden kann.

Besonders bevorzugt wird vor der Konfiguration eines neuen Anlagenmoduls aus einzelnen Bauteilkomponenten geprüft, ob ein die prozessspezifischen Anforderungen erfüllendes Anlagenmodul in der Moduldatenbank verfügbar ist. Auf diese Weise wird ein besonders ökonomisches Verfahren zur Auswahl eines die prozessspezifischen technischen Anforderungen erfüllenden Anlagenmoduls bereitgestellt.

Grundsätzlich kann das erste Serversystem ein von dem zweiten Serversystem unterschiedliches Serversystem sein, wobei das erste Serversystem mit dem zweiten Serversystem kommunikationstechnisch verbunden ist. Eine bevorzugte Weiterentwicklung der Erfindung sieht jedoch vor, dass das zweite Serversystem integraler Bestandteil des ersten Serversystems ist. Somit sind die Moduldatenbank und die Komponentendatenbank auf einem Serversystem, vorzugsweise auf dem ersten Server, angeordnet.

Die Moduldatenbank kann grundsätzlich von der Komponentendatenbank getrennt ausgebildet sein. Dies bedeutet, dass keine Verbindung zwischen der Moduldatenbank und der Komponentendatenbank vorliegt. Auf diese Weise sind die erste Eingabe der prozessspezifischen technischen Anforderungen an das Anlagenmodul in die Abfragemaske der Moduldatenbank und die zweite Eingabe der prozessspezifischen technischen Anforderungen für die identifizierte Bauteilkomponente und/oder des technischen Parameters der Baureihenkomponente in die Abfragemaske der Komponentendatenbank manuell einzugeben. Besonders bevorzugt ist jedoch vorgesehen, dass die Komponentendatenbank mit der Moduldatenbank verknüpft ist. Bei einer Verknüpfung der Komponentendatenbank mit der Moduldatenbank kann wenigstens ein Teil der manuellen Eingabe der Abfragemaske der Moduldatenbank automatisch in die Abfragemaske der Komponentendatenbank übertragen werden. Eine ganz besonders bevorzugte Weiterentwicklung der Erfindung sieht vor, dass die Komponentendatenbank integraler Bestandteil der Moduldatenbank ist. Auf diese Weise kann durch die manuelle Eingabe der prozessspezifischen technischen Anforderungen an das Anlagenmodul in die Abfragemaske der Moduldatenbank bei der Ausgabe eines die prozessspezifischen technischen Anforderungen nicht erfüllendes Anlagenmodul und bei der Identifikation der die Anforderungen an das Anlagenmodul nicht erfüllenden Bauteilkomponente eine automatische zweite Eingabe der prozessspezifischen technischen Anforderungen für die identifizierte Bauteilkomponente in die Abfragemaske der Komponentenbank erfolgen. Somit kann der Vorgang zur Konfiguration einer zur Durchführung von wenigstens einer chemischen Reaktion eingerichteten Produktionsanlage beschleunigt werden. Durch die automatische Übertragung bzw. Eingabe in die Abfragemaske der Komponentendatenbank können zudem Eingabefehler und/oder Übertragungsfehler reduziert werden.

Eine weitere vorteilhafte Weiterentwicklung der Erfindung sieht vor, dass der Vielzahl der in der Moduldatenbank hinterlegten Anlagenmodulen eine Mehrzahl die prozessspezifische Eigenschaft des jeweiligen Anlagenmoduls definierender technischer Parameter zugeordnet ist und/oder der Vielzahl der in der Komponentendatenbank hinterlegten Bauteilkomponenten mit einer Mehrzahl von Baureihenkomponenten eine Mehrzahl die prozessspezifische Eigenschaft der Baureihenkomponenten definierender technischer Parameter zugeordnet ist, und beim Abgleich der prozessspezifischen Anforderungen an ein Anlagenmodul die Mehrzahl der unterschiedlichen technischen Parameter in aufeinanderfolgenden Prüfschritten mit den prozessspezifischen Anforderungen überprüft werden.

In einer weiteren bevorzugten Weiterentwicklung der Erfindung ist vorgesehen, dass wenigstens ein die prozessspezifische Eigenschaft des jeweiligen Anlagenmoduls definierender technischer Parameter, und/oder wenigstens ein die prozessspezifische Eigenschaft der Baureihenkomponenten definierender technischer Parameter ein hartes Prüfkriterium oder ein weiches Prüfkriterium darstellt, wobei beim Abgleich der prozessspezifischen Anforderungen an das Anlagenmodul diejenigen technischen Parameter die eine hartes Prüfkriterium darstellen denjenigen technischen Parametern die ein weiches Prüfkriterium darstellen vorgezogen werden. Ein hartes Prüfkriterium betrifft vorzugsweise diejenigen technischen Parameter einer Bauteilkomponente bzw. einer Baureihenkomponente einer Bauteilkomponente die zwingend erfüllt sein müssen, um die prozessspezifischen Anforderungen zu erfüllen und/oder betrifft eine Bauteilkomponente die nur mit sehr hohem Aufwand ersetzt werden kann. Durch das vorgezogene Prüfen der harten Prüfkriterien können besonders effektiv Anlagenmodule identifiziert werden, bei denen mit geringem Aufwand eine Bauteilkomponente ausgetauscht werden kann, um ein die prozessspezifischen Anforderungen erfüllendes Anlagenmodul bereitzustellen.

Gemäß einer weiteren vorteilhaften Weiterentwicklung der Erfindung ist vorgesehen, dass die prozessspezifischen technischen Anforderungen durch einen prozessspezifischen Parametergrenzwert, dessen Über- oder Unterschreitung zu einem Erfüllen der prozesstechnischen Anforderung führt, oder durch einen prozessspezifischen Parameterbereich, dessen Einhaltung zu einem Erfüllen der prozesstechnischen Anforderung führt, definiert wird.

Eine weitere vorteilhafte Weiterentwicklung der Erfindung liegt darin, dass die prozessspezifischen technischen Anforderungen berücksichtigen, ob
- ein zur Durchführung einer bestimmten chemischen Reaktion erforderlicher Massendurchsatz in einem Anlagenmodul und/oder in einer Bauteilkomponente eines Anlagenmoduls möglich ist, und/oder
- eine Verweilzeit in einem Anlagenmodul und/oder in einer Bauteilkomponente eines Anlagenmoduls zur Durchführung einer bestimmten chemischen Reaktion zulässig ist, und/oder
- ein Betriebsdruck und/oder ein Druckverlust eines Anlagenmoduls und/oder einer Bauteilkomponente eines Anlagenmoduls zur Durchführung einer bestimmten chemischen Reaktion zulässig ist, und/oder
- eine Wärmeübertragungsleistung eines Anlagenmoduls und/oder einer Bauteilkomponente eines Anlagenmoduls zur Durchführung einer bestimmten chemischen Reaktion zulässig ist, und/oder
- geeignete Kinetiken von Reaktion und Vermischung in einem Anlagenmodul und/oder in einer Bauteilkomponente eines Anlagenmoduls während einer Durchführung einer bestimmten chemischen Reaktion vorliegen, und/oder
- ein thermisch sicherer Betrieb eines Anlagenmoduls und/oder einer Bauteilkomponente eines Anlagenmoduls während einer Durchführung einer bestimmten chemischen Reaktion gewährleistet ist, und/oder
- eine Temperatur an einem Hotspot eines Anlagenmoduls und/oder einer Bauteilkomponente eines Anlagenmoduls zur Durchführung einer bestimmten chemischen Reaktion zulässig ist, und/oder
- eine ausreichend intensive Initialvermischung von Edukten mittels eines Anlagenmoduls und/oder einer Bauteilkomponente eines Anlagenmoduls während der Durchführung einer bestimmten chemischen Reaktion erreichbar ist, und/oder
- für eine Verwendung eines Stoffes zur Durchführung von wenigstens einer chemischen Reaktion in einem Anlagenmodul und/oder in einer Bauteilkomponente eine Gefahr, wie vorzugsweise eine thermische Zersetzung und/oder eine oxidative Selbstentzündung und/oder ein Brand und/oder die Bildung einer explosionsfähigen Atmosphäre und/oder einer Gesundheitsgefährdung vorliegt.

Welche dieser prozessspezifischen technischen Anforderungen berücksichtigt werden, hängt von den jeweiligen Gegebenheiten, Anforderungen und technischen Ausgestaltungen der Anlagenmodule ab.

Die Erfindung betrifft zudem ein Computerprogramm mit auf einem computerlesbaren Datenträger gespeicherten Programmcodemitteln, die einen Computer und/oder Server und/oder eine entsprechende Recheneinheit veranlassen, ein Verfahren gemäß einer der vorgenannten Ausgestaltungen oder einer beliebigen Kombination derselben durchzuführen, wenn sie auf dem Computer bzw. der entsprechende Recheneinheit ausgeführt werden. Mit diesem Computerprogramm sind die oben mit Bezug auf das Verfahren genannten Vorteile entsprechend verbunden.

Weiterhin betrifft die Erfindung einen Datenträger mit einem vorgenannten Computerprogramm. Mit diesem Datenträger sind die vorstehend mit Bezug auf das Verfahren bzw. das Computerprogramm genannten Vorteile entsprechend verbunden.

Ferner betrifft die Erfindung ein Computersystem, auf dem ein vorgenanntes Computerprogramm geladen ist, Mit diesem Computersystem sind die vorstehend mit Bezug auf das Verfahren bzw. das Computerprogramm genannten Vorteile entsprechend verbunden.

Im Folgenden wird die Erfindung unter Bezugnahme auf die anliegenden Figuren anhand von bevorzugten Ausführungsbeispielen exemplarisch erläutert, wobei die nachfolgend dargestellten Merkmale sowohl jeweils für sich genommen als auch in Kombination miteinander einen Aspekt der Erfindung darstellen können. Es zeigen
- Fig. 1:: eine Moduldatenbank gemäß einem bevorzugten Ausführungsbeispiel der Erfindung,
- Fig. 2:: eine Komponentendatenbank gemäß dem bevorzugten Ausführungsbeispiel der Erfindung, und
- Fig. 3:: ein Verfahren zur Konfiguration einer zur Durchführung von wenigstens einer chemischen Reaktion eingerichteten Produktionsanlage gemäß dem bevorzugten Ausführungsbeispiel der Erfindung.

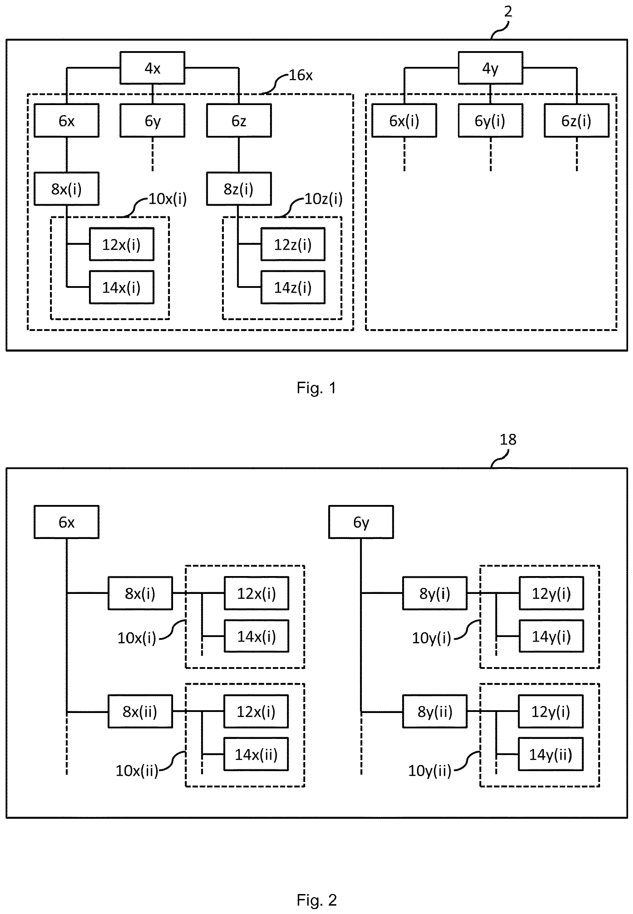

Figur 1 zeigt eine Moduldatenbank 2, die auf einem ersten Serversystem installiert ist. Die Moduldatenbank 2 umfasst eine Vielzahl von Anlagenmodulen 4x, 4y, wobei jedes Anlagenmodul 4x, 4y eine Mehrzahl von Bauteilkomponenten 6x, 6y, 6z, 6x(i), 6x(i), 6z(i) aufweist. Jede Bauteilkomponente 6x, 6y, 6z entspricht einer Baureihenkomponente 8x(i), wobei jede Baureihenkomponente 8x(i) mehrere eine prozessspezifische Eigenschaft der Baureihenkomponente 10x(i) definierende technische Parameter 12x(i), 14x(i) aufweist. Im vorliegenden Fall ist ein erster technischer Parameter 12x(i) eine bauteilkomponentenspezifische Größe der Bauteilkomponente 6x und der zweite technische Parameter 14x(i) eine baureihenspezifische Größe der Baureihenkomponente 8x(i). Eine prozessspezifische Eigenschaft eines Anlagenmoduls 16x umfasst somit mehrere die prozessspezifische Eigenschaft einer jeweiligen Baureihenkomponente 10x(i) bis 10z(i) definierende technische Parameter.

An einem konkreten Beispiel bedeutet dies, dass die Bauteilkomponente 6x des Anlagenmoduls 4x eine Pumpe ist. Die Baureihenkomponente 8x(i) der Pumpe ist dann vorzugsweise eine Membranpumpe mit einer Förderleistung A. Die prozessspezifische Eigenschaft der Membranpumpe 10x(i) wird durch die der Membranpumpe zugewiesenen technischen Parameter definiert, wobei der erste technische Parameter 12x(i) eine bauteilkomponentenspezifische Größe der Pumpe und der zweite technische Parameter 14x(i) eine baureihenspezifische Größe der Membranpumpe ist.

Figur 2 zeigt eine Komponentendatenbank 18, die auf einem zweiten Serversystem installiert ist. Die Komponentendatenbank 18 weist eine Vielzahl von Bauteilkomponenten 6x, 6y auf, wobei jede Bauteilkomponente 6x, 6y mehrere Baureihenkomponenten 8x(i), 8x(ii), 8y(i), 8y(ii) umfasst. Jeder Baureihenkomponente 8x(i), 8x(ii) sind mehrere die prozessspezifische Eigenschaft einer jeweiligen Baureihenkomponente 10x(i), 10x(ii) definierende technische Parameter zugeordnet. Die Komponentendatenbank 18 umfasst somit unterschiedliche Bauteilkomponenten 6x, 6y mit jeweils unterschiedlichen Baureihenkomponenten 8x(i), 8x(ii), 8y(i), 8y(ii).

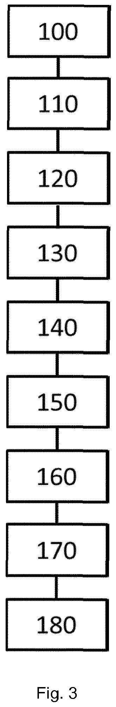

In Figur 3 ist ein Verfahren zur Konfiguration einer zur Durchführung von wenigstens einer chemischen Reaktion eingerichteten Produktionsanlage gezeigt. Die Produktionsanlage setzt sich aus mehreren Anlagenmodulen, vorzugsweise aus Reaktionsmodulen und/oder aus Lagermodulen zusammen, wobei vorliegend zur Konfiguration der Produktionsanlage ein Reaktionsmodul aus der auf dem ersten Serversystem laufenden Moduldatenbank ausgewählt bzw. aus der auf dem zweiten Serversystem laufenden Komponentendatenbank zusammengestellt wird.

Vorliegend ist das zweite Serversystem integraler Bestandteil des ersten Serversystems, so dass die Moduldatenbank und die Komponentendatenbank auf einem Serversystem laufen. Zudem sind die Komponentendatenbank und die Moduldatenbank miteinander verknüpft.

In einem ersten Verfahrensschritt 100 erfolgt zunächst eine manuelle erste Eingabe prozessspezifischer technischer Anforderungen an das Reaktionsmodul an einem mit dem Serversystem kommunikationstechnisch verbundenem Eingabemodul, wobei das Eingabemodul ein Computer ist, in eine Abfragemaske der Moduldatenbank und eine Bestätigung der Eingabe. Die prozessspezifischen technischen Anforderungen an das Reaktionsmodul sind technische Parameter, wie vorzugsweise Angaben zum Durchsatz, zum maximal und/oder minimal erlaubten Betriebsdruck, zum maximal und/oder minimal erlaubten Betriebstemperatur sowie zur minimal benötigten und/oder maximal erlaubten Verweilzeit. Auf diese Weise können Parametergrenzwerte und Parameterbereiche angegeben werden, um die prozessspezifischen technischen Anforderungen an das Reaktionsmodul zu definieren.

In einem zweiten Verfahrensschritt 110 erfolgt ein Abgleich der ersten Eingabe mit den in der Moduldatenbank hinterlegten, die prozessspezifische Eigenschaft eines jeweiligen Reaktionsmoduls definierenden, technischen Parametern. Hierbei wird überprüft, ob in der Moduldatenbank ein Reaktionsmodul verfügbar ist, das die prozessspezifischen technischen Anforderungen an das Reaktionsmodul zur Konfiguration der Produktionsanlage erfüllt. Die prozessspezifischen Eigenschaften eines jeweiligen Reaktionsmoduls werden durch die jeweilige prozessspezifische Eigenschaft der Baureihenkomponente der unterschiedlichen Bauteilkomponenten eines Reaktionsmoduls definiert.

Die unterschiedlichen Bauteilkomponenten eines in der Moduldatenbank hinterlegten Reaktionsmoduls weisen zudem unterschiedliche Prioritäten auf. Bauteilkomponenten, die einen großen Einfluss auf eine Energiebilanz und/oder Massenbilanz des Reaktionsmoduls haben, haben eine höhere Priorität als Komponenten mit niedrigen Einflüssen. Bauteilkomponenten mit einer hohen Priorisierung sind vorzugsweise ein Hauptapparat und/oder eine Fördereinrichtung. Bauteilkomponenten mit einer niedrigen Priorisierung sind vorzugsweise Komponenten der Sensorik und/oder Aktorik und/oder der Nahverrohrung.

In einem dritten Verfahrensschritt 120 wird bei einem positiven Abgleich wenigsten ein Reaktionsmodul ausgewiesen, dessen prozessspezifische Eigenschaft die prozessspezifischen technischen Anforderungen erfüllt. Bei einem negativen Abgleich, bei dem kein die prozessspezifischen technischen Anforderungen erfüllendes Reaktionsmodul in der Moduldatenbank ermittelt werden kann, wird ein die prozessspezifischen technischen Anforderungen nicht erfüllendes Reaktionsmodul in einer Ausgabemaske der Moduldatenbank ausgewiesen. Hierbei erfolgt die Auswahl des Reaktionsmoduls basierend auf der Priorisierung der Bauteilkomponenten, so dass ein Reaktionsmodul ausgewiesen wird, dessen Bauteilkomponenten mit einer hohen Priorisierung die prozessspezifischen technischen Anforderungen des Reaktionsmodels erfüllen und wenigstens eine Bauteilkomponente mit einer niedrigen Priorität die prozessspezifischen technischen Anforderungen des Reaktionsmodels nicht erfüllt.

Die die Anforderungen an das Reaktionsmodul nicht erfüllende Bauteilkomponente des ausgewiesenen Reaktionsmoduls wird identifiziert und ein die Anforderungen an das Reaktionsmoduls nicht erfüllender technischer Parameter der Baureihenkomponente der Bauteilkomponente des ausgewiesenen Reaktionsmoduls wird in der Ausgabemaske der Moduldatenbank ausgewiesen.

In einem vierten Verfahrensschritt 130 erfolgt eine manuelle zweite Eingabe prozessspezifischer technischer Anforderungen für die identifizierte Bauteilkomponente an dem Computer in eine Abfragemaske der Komponentendatenbank und eine Bestätigung der Eingabe.

In einem fünften Verfahrensschritt 140 erfolgt ein Abgleich der zweiten Eingabe mit den in der Komponentendatenbank hinterlegten, die prozessspezifische Eigenschaft einer jeweiligen Baureihenkomponente definierenden, technischen Parametern der Vielzahl von Baureihenkomponenten.

In einem sechsten Verfahrensschritt 150 wird bei einem positiven Abgleich wenigstens eine die prozessspezifischen technischen Anforderungen erfüllende Baureihenkomponente einer Bauteilkomponente ausgewiesen. Auf diese Weise kann das die prozessspezifischen Anforderungen nicht erfüllende Reaktionsmodul mit der ausgewiesenen Baureihenkomponente konfiguriert werden, um ein die prozessspezifischen Anforderungen erfüllendes Reaktionsmodul bereitzustellen.

Bei einem negativem Abgleich erfolgt in einem siebten Verfahrensschritt 160 eine manuelle dritte Eingabe der prozessspezifischen technischen Anforderungen an das Reaktionsmodul an dem Computer in die Abfragemaske der Komponentendatenbank.

In einem achten Verfahrensschritt 170 erfolgt ein Abgleich der prozessspezifischen technischen Anforderungen an das Reaktionsmodul mit den in der Komponentendatenbank hinterlegten, die prozessspezifische Eigenschaft einer jeweiligen Baureihenkomponente definierenden, technischen Parameter der Vielzahl von Baureihenkomponenten der unterschiedlichen Bauteilkomponenten.

In einem neunten Verfahrensschritt 180 erfolgt die Ausgabe einer Vielzahl von Bauteilkomponenten, dessen prozessspezifische Eigenschaften der Baureihenkomponenten die prozessspezifischen technischen Anforderungen an das Reaktionsmodul erfüllen, auf der Ausgabemaske der Komponentendatenbank. Zudem wird ein die prozessspezifischen technischen Anforderungen erfüllendes Reaktionsmodul aus der Vielzahl der ausgegeben Bauteilkomponenten und/oder Baureihenkomponenten bereitgestellt.

Auf diese Weise wird ein Verfahren bereitgestellt, bei dem zunächst überprüft wird, ob ein Reaktionsmodul aus dem Bestand den technischen Anforderungen eines Reaktionsmoduls zur Konfiguration einer neuen Produktionsanlage entspricht. Sofern dies nicht der Fall ist, wird überprüft, ob ein bereits vorhandenes Reaktionsmodul mit geringem Aufwand, vorzugsweise durch den Austausch einer Bauteilkomponente, umgebaut werden könnte, um auf diese Weise ein die prozessspezifischen technischen Anforderungen erfüllendes Reaktionsmodul bereitzustellen. Sofern auch dies nicht möglich ist, wird ein neues Reaktionsmodul mit Bauteilkomponenten aus der Komponentendatenbank bereitgestellt.

Auf diese Weise wird ein Verfahren bereitgestellt, das eine zügige und preiswerte Konfiguration einer zur Durchführung von wenigstens einer chemischen Reaktion eingerichteten Produktionsanlage ermöglicht.

Bezugszeichen (ausgenommen Bezugszeichen ab Nummer 100, welche Verfahrensschritte abbilden):
- 2: Moduldatenbank
- 4: Anlagenmodul
- 6: Bauteilkomponente
- 8: Baureihenkomponente
- 10: Prozessspezifische Eigenschaft der Baureihenkomponente
- 12: Erster technischer Parameter
- 14: Zweiter technischer Parameter
- 16: Prozessspezifische Eigenschaft des Anlagenmoduls
- 18: Komponentendatenbank

## Patentansprüche

1. Verfahren zur Konfiguration einer zur Durchführung von wenigstens einer chemischen Reaktion eingerichteten Produktionsanlage, wobei
die Produktionsanlage wenigstens ein Anlagenmodul (4x, 4y) aufweist, und zur Konfiguration der Produktionsanlage das Anlagenmodul (4x, 4y) aus einer auf einem ersten Serversystem laufenden Moduldatenbank (2) ausgewählt und aus einer auf dem ersten Serversystem laufenden Komponentendatenbank zusammengestellt wird oder das Anlagenmodul (4x, 4y) aus einer auf einem ersten Serversystem laufenden Moduldatenbank (2) ausgewählt und aus einer auf einem zweiten Serversystem laufenden Komponentendatenbank (18) zusammengestellt wird; **dadurch gekennzeichnet, dass** das Auswählen oder Zusammenstellen des Anlagenmoduls (4x, 4y) folgende Schritte umfasst:
- Manuelle erste Eingabe (100) prozessspezifischer technischer Anforderungen an das Anlagenmodul (4x, 4y) in eine Abfragemaske der Moduldatenbank (2) und Bestätigung der Eingabe;
- Abgleich (110) der ersten Eingabe mit wenigstens einem in der Moduldatenbank hinterlegten, eine prozessspezifische Eigenschaft eines jeweiligen Anlagenmoduls definierenden, technischen Parameter einer Vielzahl von Anlagenmodulen auf dem ersten Serversystem, zum Bestimmen, ob in der Moduldatenbank (2) ein Anlagenmodul verfügbar ist, das die prozessspezifischen technischen Anforderungen an das Anlagenmodul (4x, 4y) zur Konfiguration der Produktionsanlage erfüllt, und bei einem negativem Ergebnis:
(i) Ausweisen (120) wenigstens eines Anlagenmoduls, dessen prozessspezifische Eigenschaft die prozessspezifischen technischen Anforderungen nicht erfüllt, in einer Ausgabemaske der Moduldatenbank;
(ii) Identifikation wenigstens einer die Anforderungen an das Anlagenmodul (4x, 4y) nicht erfüllenden Bauteilkomponente des ausgewiesenen Anlagenmoduls und/oder eines die Anforderungen an das Anlagenmodul (4x, 4y) nicht erfüllenden technischen Parameters einer Baureihenkomponente der Bauteilkomponente des ausgewiesenen Anlagenmoduls;
- Manuelle oder automatische zweite Eingabe (130) prozessspezifischer technischer Anforderungen für die identifizierte Bauteilkomponente und/oder des technischen Parameters der Baureihenkomponente in eine Abfragemaske der Komponentendatenbank (18);
- Abgleich (140) der zweiten Eingabe mit wenigstens einem in der Komponentendatenbank (18) hinterlegten, eine prozessspezifische Eigenschaft einer jeweiligen Baureihenkomponente definierenden, technischen Parameter einer Vielzahl von Baureihenkomponenten, und bei positivem Ergebnis:
(a) Ausweisen (150) wenigstens einer die prozessspezifischen technischen Anforderungen erfüllenden Baureihenkomponente einer Bauteilkomponente in einer Ausgabemaske der Komponentendatenbank (18) und Zusammenstellen des ausgegeben Anlagenmoduls mit der ausgegebenen Baureihenkomponente.

2. Verfahren nach Anspruch 1, wobei nach einem positiven Abgleich (110) prozessspezifischer technischer Anforderungen an das Anlagenmodul (4x, 4y) mit den in der Moduldatenbank (2) hinterlegten technischen Parametern der Vielzahl von Anlagenmodulen der folgende Schritt erfolgt:
- Ausweisen wenigstens eines Anlagenmoduls, dessen prozessspezifische Eigenschaft die prozessspezifischen technischen Anforderungen erfüllt.

3. Verfahren nach einem der Ansprüche 1 oder 2, wobei nach einem negativen Abgleich der manuellen oder automatischen Eingabe (130) der prozessspezifischen technischen Anforderungen für die identifizierte Bauteilkomponente und/oder des technischen Parameters der Baureihenkomponente mit den in der Komponentendatenbank hinterlegten technischen Parameter der Vielzahl von Baureihenkomponenten die folgenden Schritte erfolgen:
- Manuelle oder automatische dritte Eingabe (160) prozessspezifischer technischer Anforderungen an das Anlagenmodul (4x, 4y) in die Abfragemaske der Komponentendatenbank (18);
- Abgleich (170) prozessspezifischer technischer Anforderungen an das Anlagenmodul (4x, 4y) mit den in der Komponentendatenbank (18) hinterlegten, die prozessspezifische Eigenschaft einer jeweiligen Baureihenkomponente definierenden, technischen Parameter der Vielzahl von Baureihenkomponenten der Bauteilkomponenten;
- Ausgabe (180) einer Vielzahl von Bauteilkomponenten, dessen prozessspezifische Eigenschaften der Baureihenkomponenten die prozessspezifischen technischen Anforderungen an das Anlagenmodul (4x, 4y) erfüllen; und
- Bereitstellen eines die prozessspezifischen technischen Anforderungen erfüllendes Anlagenmodul aus der Vielzahl der ausgegeben Bauteilkomponenten und/oder Baureihenkomponenten.

4. Verfahren nach einem der vorhergehenden Ansprüche, wobei wenigstens ein eine prozessspezifische Eigenschaft des jeweiligen Anlagenmoduls definierender technischer Parameter (16x), und/oder wenigstens ein eine prozessspezifische Eigenschaft der Baureihenkomponenten definierender technischer Parameter (10x(i), 10z(i)) ein hartes Prüfkriterium oder ein weiches Prüfkriterium darstellt, wobei beim Abgleich der prozessspezifischen Anforderungen an das Anlagenmodul (4x, 4y) diejenigen technischen Parameter, die eine hartes Prüfkriterium darstellen, denjenigen technischen Parametern, die ein weiches Prüfkriterium darstellen, vorgezogen werden.

5. Verfahren nach einem der vorhergehenden Ansprüche, wobei der Vielzahl der in der Moduldatenbank (2) hinterlegten Anlagenmodule (4x) eine Mehrzahl die prozessspezifischen Eigenschaften des jeweiligen Anlagenmoduls definierende technische Parameter (16x) zugeordnet ist und/oder der Vielzahl der in der Komponentendatenbank (18) hinterlegten Bauteilkomponenten (6x, 6y) mit einer Mehrzahl von Baureihenkomponenten (8x(i), 8x(ii), 8y(i), 8y(ii)) eine Mehrzahl die prozessspezifische Eigenschaft der Baureihenkomponenten definierender technischer Parameter (10x(i), 10x(ii), 10y(i), 10y(ii)) zugeordnet ist, und beim Abgleich der prozessspezifischen Anforderungen an das Anlagenmodul (4x, 4y) die Mehrzahl der unterschiedlichen technischen Parameter in aufeinanderfolgenden Prüfschritten mit den prozessspezifischen Anforderungen überprüft werden.

6. Verfahren gemäß einem der vorhergehenden Ansprüche, wobei die prozessspezifischen technischen Anforderungen durch einen prozessspezifischen Parametergrenzwert, dessen Über- oder Unterschreitung zu einem Erfüllen der prozesstechnischen Anforderung führt, oder durch einen prozessspezifischen Parameterbereich, dessen Einhaltung zu einem Erfüllen der prozesstechnischen Anforderung führt, definiert wird.

7. Verfahren gemäß einem der vorhergehenden Ansprüche, wobei die prozessspezifischen technischen Anforderungen berücksichtigen, ob
- ein zur Durchführung einer bestimmten chemischen Reaktion erforderlicher Massendurchsatz in dem Anlagenmodul (4x, 4y) oder in einer Bauteilkomponente des Anlagenmoduls (4x, 4y) möglich ist, und/oder
- eine Verweilzeit in dem Anlagenmodul (4x, 4y) oder in einer Bauteilkomponente des Anlagenmoduls (4x, 4y) zur Durchführung einer bestimmten chemischen Reaktion zulässig ist, und/oder
- ein Betriebsdruck und/oder ein Druckverlust des Anlagenmoduls (4x, 4y) oder einer Bauteilkomponente des Anlagenmoduls (4x, 4y) zur Durchführung einer bestimmten chemischen Reaktion zulässig ist, und/oder
- eine Wärmeübertragungsleistung des Anlagenmoduls (4x, 4y) oder einer Bauteilkomponente des Anlagenmoduls (4x, 4y) zur Durchführung einer bestimmten chemischen Reaktion zulässig ist, und/oder
- geeignete Kinetiken von Reaktion und Vermischung in dem Anlagenmodul (4x, 4y) oder in einer Bauteilkomponente des Anlagenmoduls (4x, 4y) während einer Durchführung einer bestimmten chemischen Reaktion vorliegen, und/oder
- ein thermisch sicherer Betrieb des Anlagenmoduls (4x, 4y) oder einer Bauteilkomponente des Anlagenmoduls (4x, 4y) während einer Durchführung einer bestimmten chemischen Reaktion gewährleistet ist, und/oder
- eine Temperatur an einem Hotspot des Anlagenmoduls (4x, 4y) oder einer Bauteilkomponente des Anlagenmoduls (4x, 4y) zur Durchführung einer bestimmten chemischen Reaktion zulässig ist, und/oder
- eine ausreichend intensive Initialvermischung von Edukten mittels des Anlagenmoduls (4x, 4y) oder einer Bauteilkomponente des Anlagenmoduls (4x, 4y) während der Durchführung einer bestimmten chemischen Reaktion erreichbar ist, und/oder
- für eine Verwendung eines Stoffes zur Durchführung von wenigstens einer chemischen Reaktion in dem Anlagenmodul (4x, 4y) oder in einer Bauteilkomponente eine Gefahr, wie vorzugsweise eine thermische Zersetzung und/oder eine oxidative Selbstentzündung und/oder ein Brand und/oder die Bildung einer explosionsfähigen Atmosphäre und/oder einer Gesundheitsgefährdung, vorliegt.

8. Computerprogramm mit auf einem computerlesbaren Datenträger gespeicherten Programmcodemitteln, die einen Computer oder eine entsprechenden Recheneinheit veranlassen, ein Verfahren gemäß einem der vorhergehenden Ansprüche durchzuführen, wenn sie auf dem Computer bzw. der entsprechenden Recheneinheit ausgeführt werden.

9. Datenträger mit einem Computerprogramm nach Anspruch 8.

## Claims

1. A method for configuration of a production plant set up for carrying out at least one chemical reaction, wherein
the production plant comprises at least one plant module (4x, 4y), and, for configuration of the production plant, the plant module (4x, 4y) is selected from a module database (2) running on a first server system and is compiled from a component database running on the first server system, or the plant module (4x, 4y) is selected from a module database (2) running on a first server system and is compiled from a component database (18) running on a second server system; **characterised in that** the selection or compilation of the plant module (4x, 4y) comprises the following steps:
- manual first input (100) of process-specific technical requirements for the plant module (4x, 4y) in a query mask of the module database (2) and confirmation of the input;
- comparing (110) the first input with at least one technical parameter, stored in the module database and defining a process-specific property of a respective plant module, of a plurality of plant modules on the first server system, for determining whether a plant module is available in the module database (2) which fulfils the process-specific technical requirements for the plant module (4x, 4y) for configuration of the production plant, and if the result is negative:
(i) designating (120) at least one plant module whose process-specific property does not fulfil the process-specific technical requirements in an output mask of the module database;
(ii) identifying at least one module component of the designated plant module which does not fulfil the requirements for the plant module (4x, 4y) and/or of a technical parameter of a series component of the module component of the designated plant module which does not fulfil the requirements for the plant module (4x, 4y);
- manual or automatic second input (130) of process-specific technical requirements for the identified module component and/or the technical parameter of the series component into a query mask of the component database (18);
- comparing (140) the second input with at least one technical parameter of a plurality of series components stored in the component database (18) and defining a process-specific property of a respective series component, and if the result is positive:
(a) designating (150) at least one series component of a module component which fulfils the process-specific technical requirements in an output mask of the component database (18) and compiling the output plant module with the output series component.

2. The method according to claim 1, wherein, after a positive comparison (110) of process-specific technical requirements for the plant module (4x, 4y) with the technical parameters of the plurality of plant modules stored in the module database (2), the following step takes place:
- designating at least one plant module whose process-specific property fulfils the process-specific technical requirements.

3. The method according to any one of claims 1 or 2, wherein after a negative comparison of the manual or automatic input (130) of the process-specific technical requirements for the identified module component and/or the technical parameter of the series component with the technical parameters of the plurality of series components stored in the component database, the following steps are performed:
- manual or automatic third input (160) of process-specific technical requirements for the plant module (4x, 4y) in the query mask of the component database (18);
- comparing (170) process-specific technical requirements for the plant module (4x, 4y) with the technical parameters of the plurality of series components of the module components stored in the component database (18) and defining the process-specific property of a respective series component;
- output (180) of a plurality of module components whose process-specific properties of the series components fulfil the process-specific technical requirements for the plant module (4x, 4y); and
- provision of a plant module that fulfils the process-specific technical requirements from the plurality of output module components and/or series components.

4. The method according to any one of the preceding claims, wherein at least one technical parameter (16x) defining a process-specific property of the respective plant module and/or at least one technical parameter (10x(i), 10z(i)) defining a process-specific property of the series components represents a hard test criterion or a soft test criterion, wherein, when comparing the process-specific requirements for the plant module (4x, 4y), those technical parameters which represent a hard test criterion are given preference over those technical parameters which represent a soft test criterion.

5. The method according to any one of the preceding claims, wherein the plurality of plant modules (4x) stored in the module database (2) is assigned a plurality of technical parameters (16x) defining the process-specific properties of the respective plant module and/or the plurality of module components (6x, 6y) stored in the component database (18) is assigned a plurality of series components (8x(i), 8x(ii), 8y(i), 8y(ii)) stored in the component database (18) is assigned a plurality of technical parameters (10x(i), 10x(ii), 10y(i), 10y(ii)) defining the process-specific property of the series components, and when comparing the process-specific requirements for the plant module (4x, 4y), the plurality of different technical parameters are checked in successive test steps against the process-specific requirements.

6. Method according to one of the preceding claims, wherein the process-specific technical requirements are defined by a process-specific parameter limit value, the exceeding or falling below of which leads to fulfilment of the process-specific technical requirement, or by a process-specific parameter range, compliance with which leads to fulfilment of the process-specific technical requirement.

7. Method according to any one of the preceding claims, wherein the process-specific technical requirements take into account whether
- a mass flow rate required for carrying out a specific chemical reaction is possible in the plant module (4x, 4y) or in a module component of the plant module (4x, 4y), and/or
- a dwell time in the plant module (4x, 4y) or in a module component of the plant module (4x, 4y) is permissible for carrying out a specific chemical reaction, and/or
- an operating pressure and/or a pressure loss of the plant module (4x, 4y) or of a module component of the plant module (4x, 4y) is permissible for carrying out a specific chemical reaction, and/or
- a heat transfer capacity of the plant module (4x, 4y) or a module component of the plant module (4x, 4y) is permissible for carrying out a specific chemical reaction, and/or
- suitable kinetics of reaction and mixing are present in the plant module (4x, 4y) or in a module component of the plant module (4x, 4y) during performance of a specific chemical reaction, and/or
- thermally safe operation of the plant module (4x, 4y) or a module component of the plant module (4x, 4y) is ensured during the performance of a specific chemical reaction, and/or
- a temperature at a hotspot of the plant module (4x, 4y) or a module component of the plant module (4x, 4y) is permissible for carrying out a specific chemical reaction, and/or
- a sufficiently intensive initial mixing of educts can be achieved by means of the plant module (4x, 4y) or a module component of the plant module (4x, 4y) during performance of a specific chemical reaction, and/or
- a hazard, such as preferably thermal decomposition and/or oxidative spontaneous combustion and/or fire and/or the formation of an explosive atmosphere and/or a health hazard, is present for the use of a substance for carrying out at least one chemical reaction in the plant module (4x, 4y) or in a module component.

8. A computer program comprising program code stored on a computerreadable data carrier, which causes a computer or a corresponding computing unit to perform a method according to any one of the preceding claims when executed on the computer or the corresponding computing unit.

9. A data carrier with a computer program according to claim 8.

## Revendications

1. Procédé pour la configuration d'une installation de production conçue pour le déroulement d'au moins une réaction chimique, dans lequel l'installation de production comprend au moins un module d'installation (4x, 4y) et, pour la configuration de l'installation de production, le module d'installation (4x, 4y) est sélectionné dans une base de données de modules (2) tournant sur un premier système de serveur et est assemblé à partir d'une base de données de composants tournant sur le premier système de serveur ou le module d'installation (4x, 4y) est sélectionné dans une base de données de modules (2) tournant sur un premier système de serveur et est assemblé à partir d'une base de données de composants (18) tournant sur un deuxième système de serveur ;
**caractérisé en ce que** la sélection ou l'assemblage du module d'installation (4x, 4y) comprend les étapes suivantes :
- première entrée manuelle (100) d'exigences techniques spécifiques au processus concernant le module d'installation (4x, 4y) dans un masque de requête de la base de données de modules (2) et confirmation de l'entrée ;
- comparaison (110) de la première entrée avec au moins un paramètre technique, enregistré dans la base de données de modules, définissant une propriété, spécifique au processus, d'un module d'installation respectif, d'une pluralité de modules d'installation sur le premier système de serveur, afin de déterminer si, dans la base de données de modules (2), un module d'installation est disponible, qui satisfait les exigences techniques spécifiques au processus concernant le module d'installation (4x, 4y) pour la configuration de l'installation de production et, dans le cas d'un résultat négatif :
(i) identification (120) d'au moins un module d'installation dont la propriété spécifique au processus ne satisfait pas les exigences techniques spécifiques au processus, dans un masque de sortie de la base de données de modules ;
(ii) identification d'au moins un composant d'élément de construction du module d'installation identifié ne satisfaisant pas exigences concernant le module d'installation (4x, 4y) et/ou d'un paramètre technique d'un composant de série du composant d'élément de construction du module d'installation identifié ;
- deuxième entrée manuelle ou automatique (130) d'exigences techniques spécifiques au processus pour le composant d'élément de construction identifié et/ou du paramètre technique du composant de série dans un masque de requête de la base de données de composants (18) ;
- comparaison (140) de la deuxième entrée avec au moins un paramètre technique, enregistré dans la base de données de composants (18), définissant une propriété, spécifique au processus, d'un composant de série respectif, d'une pluralité de composants de série et, dans le cas d'un résultat positif :
(a) identification (150) d'au moins un composant de série d'un composant d'élément de construction, satisfaisant les exigences techniques spécifiques au processus, dans un masque de sortie de la base de données de composants (18) et assemblage du module d'installation sortie avec le composant de série sorti.

2. Procédé selon la revendication 1, dans lequel, après une comparaison positive (110) d'exigences techniques spécifiques au processus concernant le module d'installation (4x, 4y) avec les paramètres techniques, enregistrés dans la base de données de modules (2), de la pluralité de modules d'installation, est exécutée l'étape suivante :
- identification d'au moins un module d'installation dont la propriété spécifique au processus satisfait les exigences techniques spécifiques au processus.

3. Procédé selon l'une des revendications 1 ou 2, dans lequel, après une comparaison négative de l'entrée manuelle ou automatique (130) des exigences techniques spécifiques au processus pour le composant de série identifié et/ou du paramètre technique du composant de série avec les paramètres techniques, enregistrés dans la base de données de composants, de la pluralité de composants de série, sont exécutées les étapes suivantes :
- troisième entrée manuelle ou automatique (160) d'exigences techniques spécifiques au processus concernant le module d'installation (4x, 4y) dans le masque de requête de la base de données de composants (18) ;
- comparaison (170) des exigences technique spécifiques au processus concernant le module d'installation (4x, 4y) avec les paramètres techniques, enregistrés dans la base de données de composants, définissant la propriété spécifique au processus d'un composant de série respectif, de la pluralité de composants de série des composants d'éléments de construction ;
- sortie (180) d'une pluralité de composants d'éléments de construction dont les propriétés spécifiques au processus des composants de série satisfont les exigences techniques spécifiques au processus concernant le module d'installation (4x, 4y) ; et
- mise à disposition d'un module d'installation satisfaisant les exigences techniques spécifiques au processus parmi la pluralité de composants d'éléments de construction et/ou de composants de série sortis.

4. Procédé selon l'une des revendications précédentes, dans lequel au moins un paramètre technique (16x) définissant une propriété spécifique au processus du module d'installation respectif et/ou au moins un paramètre technique (10x(i), 10z(i)) définissant une propriété spécifique au processus du composant de série représente un critère de contrôle strict ou un critère de contrôle souple, dans lequel, lors de la comparaison des exigences spécifiques au processus concernant le module d'installation (4x, 4y), les paramètres techniques qui représentent un critère de contrôle strict sont préférés aux paramètres techniques qui représentent un critère de contrôle souple.

5. Procédé selon l'une des revendications précédentes, dans lequel, à la pluralité de modules d'installation (4x) enregistrés dans la base de données de modules (2) correspond une pluralité de paramètres techniques (16x) définissant les propriétés spécifiques au processus du module d'installation respectif et/ou, à la pluralité de composants d'éléments de construction (6x, 6y) enregistrés dans la base de données de composants (18), avec une pluralité de composants de série (8x(i), 8x(ii), 8y(i), 8y(ii)), correspond une pluralité de paramètres techniques (10x(i), 10x(ii), 10y(i), 10y(ii)) définissant la propriété spécifique au processus des composants de série et, lors de la comparaison des exigences spécifiques au processus concernant le module d'installation (4x, 4y), la pluralité des différents paramètres techniques sont contrôlés dans des étapes de contrôle successives avec les exigences spécifiques au processus.

6. Procédé selon l'une des revendications précédentes, dans lequel les exigences techniques spécifiques au processus sont définies par une valeur limite de paramètre spécifique au processus, dont le dépassement ou le passage en dessous conduit à un respect de l'exigence spécifique au processus, ou par une plage de paramètres spécifique au processus, dont le respect conduit à un respect de l'exigence spécifique au processus.

7. Procédé selon l'une des revendications précédentes, dans lequel les exigences techniques spécifiques au processus vérifient si
- un débit massique nécessaire à le déroulement d'une réaction chimique déterminée est possible dans le module d'installation (4x, 4y) ou dans un composant d'élément de construction du module d'installation (4x, 4y) et/ou
- un temps de séjour dans le module d'installation (4x, 4y) ou dans un composant d'élément de construction du module d'installation (4x, 4y) est acceptable pour le déroulement d'une réaction chimique déterminée et/ou
- une pression de service et/ou une perte de pression du module d'installation (4x, 4y) ou d'un composant d'élément de construction du module d'installation (4x, 4y) est acceptable pour le déroulement d'une réaction chimique déterminée et/ou
- une puissance de transfert thermique du module d'installation (4x, 4y) ou d'un composant d'élément de construction du module d'installation (4x, 4y) est acceptable pour le déroulement d'une réaction chimique déterminée et/ou
- des cinétiques appropriées de réaction et de mélange règnent dans le module d'installation (4x, 4y) ou dans un composant d'élément de construction du module d'installation (4x, 4y) pendant le déroulement d'une réaction chimique déterminée et/ou
- un fonctionnement thermique sûr du module d'installation (4x, 4y) ou d'un composant d'élément de construction du module d'installation (4x, 4y) est garanti pendant le déroulement d'une réaction chimique déterminée et/ou
- une température au niveau d'un point chaud du module d'installation (4x, 4y) ou d'un composant d'élément de construction du module d'installation (4x, 4y) est acceptable pour le déroulement d'une réaction chimique déterminée et/ou
- un mélange initial suffisamment intensif d'éduits peut être obtenu au moyen du module d'installation (4x, 4y) ou d'un composant d'élément de construction du module d'installation (4x, 4y) pendant le déroulement d'une réaction chimique déterminée et/ou
- pour une utilisation d'un matériau pour le déroulement d'au moins une réaction chimique dans le module d'installation (4x, 4y) ou un composant d'élément de construction, il existe un risque, par exemple, de préférence, une décomposition thermique et/ou un auto-allumage oxydatif et/ou un incendie et/ou la formation d'une atmosphère explosive et/ou un risque sanitaire.

8. Programme informatique avec des moyens de codage de programme, enregistrés sur un support de données lisible par un ordinateur, qui font en sorte qu'un ordinateur ou une unité de calcul correspondante exécute un procédé selon l'une des revendications précédentes lorsqu'ils sont exécutés sur l'ordinateur ou l'unité de calcul correspondante.

9. Support de données avec un programme informatique selon la revendication 8.
